# EUROPEAN PATENT APPLICATION

(11) **EP 4 053 248 A1**
(43) Date of publication of application: **07.09.2022**
(21) Application number: 20881277.6
(22) Date of filing: 28.10.2020
(51) Int. Cl.: C10G 9/14, C10G 55/00

(54) **METHOD AND SYSTEM FOR DIRECTLY CRACKING CRUDE OIL TO PREPARE OLEFIN**

(30) Priority: 28.10.2019 CN 201911027979
(71) Applicant: CHINA PETROLEUM & CHEMICAL CORPORATION, Chaoyang District Beijing 100728 (CN); BEIJING RESEARCH INSTITUTE OF CHEMICAL INDUSTRY, CHINA PETROLEUM & CHEMICAL CORPORATION, Chaoyang District Beijing 100013 (CN)
(72) Inventor: LIU, Tongju, Beijing 100013 (CN); WANG, Guoqing, Beijing 100013 (CN); ZHANG, Lijun, Beijing 100013 (CN); ZHANG, Zhaobin, Beijing 100013 (CN); ZHOU, Cong, Beijing 100013 (CN); JIANG, Bing, Beijing 100013 (CN); SHI, Ying, Beijing 100013 (CN)
(74) Representative: Hoffmann Eitle
(86) International application number: PCT/CN2020/124197
(87) International publication number: WO 2021/083165

(57) **Abstract**

The present invention relates to a method and system for cracking crude oil. The method comprises: delivering the crude oil to a first tube group of a convection section of a cracking furnace for preheating and then performing vaporization to obtain a first gas phase and a first liquid phase; performing high-pressure extraction on the first liquid phase to obtain a non-asphalt oil and an asphalt; and mixing the first gas phase and the non-asphalt oil with water vapor respectively, or mixing the first gas phase with the non-asphalt oil prior to mixing with water vapor, then delivering the same to a second tube group of the convection section of the cracking furnace for heating, followed by delivering same to a radiation section of the cracking furnace for cracking to obtain a cracked product, and separating the cracked product to obtain low-carbon olefins. In the present invention, after the crude oil is preheated in a cracking furnace and vaporized, the obtained gas phase is passed into the cracking furnace, and the obtained liquid phase is passed through a supercritical extraction unit to separate an extract oil, which is then passed into the cracking furnace The method can effectively improve cracking efficiency of the crude oil, improve the yield of low-carbon olefins in the product, extend the operating cycle, and reduce coking of a gasified crude oil mixture in a cracking process.

## Description

### FIELD OF THE INVENTION

The present invention relates to the field of crude oil cracking, in particular to a method and system for cracking crude oil, and specifically to a method and system for directly cracking crude oil to prepare olefins.

### BACKGROUND OF THE INVENTION

Low-carbon olefins usually refer to the general term for unsaturated hydrocarbons with four or less carbon atoms, mainly including ethylene, propylene, butadiene and other organic chemical raw materials with a high economic value. With development of China's economy, the demand for these organic chemical raw materials is increasing year by year. Although production scale of low-carbon olefins is also increasing year by year, it cannot meet the increasing demand.

For a long time, China has always used naphtha as a main raw material for the preparation of low-carbon olefins. However, in recent years, with a massive exploitation of oilfield associated gas in Middle East and shale gas in the United States, these cheap oil and gas resources have been widely used as raw materials for ethylene, resulting in lower prices of ethylene-related products. In order to cope with impact of market competition, expanding a source of raw materials for an ethylene cracking apparatus and reducing cost of the raw materials have become an effective means for traditional ethylene enterprises to reduce cost and increase efficiency. Therefore, using special heavy hydrocarbons, especially unprocessed crude oil, as raw materials for a cracking furnace to produce low-carbon olefins is conducive to reducing the raw material cost and energy consumption of an olefin production apparatus, and quickly adapting to changes in the supply and demand for cracking raw materials in the market.

In order to make full use of crude oil resources and improve the yield of low-carbon olefins, a cracking furnace is usually used to crack various hydrocarbon feedstocks into olefins by vapor cracking. A commonly-used cracking furnace comprises a convection section and a radiation section. Crude oil is generally divided into four components of saturates, aromatics, colloids and asphaltenes, wherein saturates and asphaltenes represent the most stable and most unstable components in crude oil, respectively. Crude oil comprises high molecular weight non-volatile components having a boiling point exceeding 590°C. When these non-volatile components are preheated in the convection section of a conventional cracking furnace, a small part is not gasified, and the un-gasified non-volatile components are entrained into the radiation section along with the mixed gas flow, which is likely to cause coking deposition in the radiation section, or even block the radiation section, thereby affecting yield of cracked products.

CN101583697A discloses a process for cracking a synthetic oil-containing feedstock comprising: 1) hydroprocessing a wide boiling range aliquot containing a) a normally liquid hydrocarbon portion boiling in a range from 50 °F to 800 °F, substantially free of resids, and b) a thermally cracked hydrocarbon liquid boiling in a range from 600 °F to 1050 °F, to provide a synthetic crude oil boiling in a range of from 73 °F to 1070 °F, containing greater than 25 wt% aromatics, greater than 25 wt% naphthenes, less than 0.3 wt% S, less than 0.02 wt% asphaltenes, and substantially free of resids other than asphaltenes; 2) adding to the synthetic crude oil a normally liquid hydrocarbon component boiling in a range from 100 °F to 1050 °F; and 3) cracking the mixture resulting from 2) in a cracker furnace comprising a radiant coil outlet to provide a cracked effluent, wherein the cracking is carried out under conditions sufficient to effect a radiant coil outlet temperature which is greater than the optimum radiant coil outlet temperature for cracking the synthetic crude oil separately.

In said process, an existing raw material for ethylene production is mixed into the crude oil, to dilute the crude oil, improve cracking performance of the crude oil, and improve conversion of olefins. However, this process is limited by the source of the existing raw material for ethylene production, and cannot effectively utilize a large amount of crude oil for the production of low-carbon olefins.

CN1957068A discloses steam cracking of hydrocarbon feedstocks containing salts and/or particulate matters, the method comprising: a) adding an undesalted feedstock comprising salts and optional particulate matters to a convection section of a pyrolysis furnace; b) heating said hydrocarbon feedstock; c) feeding the hydrocarbon feedstock to a flash/separation vessel located upstream of a dry point; d) separating the hydrocarbon feedstock into a vapor phase substantially depleted in nonvolatile components and salts and a liquid phase enriched in nonvolatile components and salts, which liquid phase comprises 5% liquid phase at all points in the convective section upstream of the flash/separation vessel to maintain the salts and any particulate matters in suspension; e) removing 50-95% of the hydrocarbon feedstock of step a in the vapor phase from the flash/separation vessel and cracking the vapor phase to produce an effluent containing olefins; and f) removing at least 5% of the hydrocarbon feedstock in liquid phase from the flash/separation vessel along with the salt and any particulate matter in suspension. US3617493 also discloses a method for cracking crude oil with steam which is similar to the above method.

CN1041967A discloses a process for thermally cracking a low-quality feedstock containing heavy fractions in a cracking furnace, wherein said low-quality feed stock is withdrawn from a preheater of the cracking furnace to separate and remove the heavy fractions from said low-quality feed stock by a vaporization method, and thereafter is returned to said preheater before subjecting said feedstock to a thermal cracking.

The above methods of cracking a feedstock all use a flash tank to treat the crude oil passing through the convection section of the cracking furnace to achieve vaporization, and the gas phase is passed into the radiation section for cracking. However, after the feedstock is flashed, at least 5% of the hydrocarbon feedstock still remains in the liquid, which affects the yield of low-carbon olefins. Moreover, the separated liquid phase is directly used as fuel oil, resulting in waste of the feedstock.

Therefore, there is still a need for an improved method and system for cracking crude oil, which can reduce coking during cracking of the crude oil and increase the yield of low-carbon olefins.

### SUMMARY OF THE INVENTION

In order to overcome problems of insufficient crude oil vaporization, easy coking during cracking, and low yield of low-carbon olefins in the prior art, the present invention provides a method and system for cracking crude oil, and specifically relates to a method and system for directly cracking crude oil to prepare olefins, which can improve utilization rate and cracking efficiency of the crude oil feedstock, prolong operating cycle, reduce coking during cracking, and simultaneously improve the yield of low-carbon olefins in a product.

In one aspect, the present invention provides a method for cracking crude oil, comprising the following steps:
step 1. delivering the crude oil to a convection section of a cracking furnace for preheating, and then performing vaporization to obtain a first gas phase and a first liquid phase;
step 2. performing high-pressure extraction on the first liquid phase to obtain a non-asphalt oil and an asphalt;
step 3. mixing the first gas phase and the non-asphalt oil with water vapor respectively, or mixing the first gas phase with the non-asphalt oil prior to mixing with water vapor, then delivering the same to the convection section of the cracking furnace for heating to a crossover temperature, followed by delivering the same to a radiation section of the cracking furnace for cracking to obtain a cracked product, and separating the cracked product to obtain low-carbon olefins.

Specifically, the present invention provides a method for cracking crude oil, comprising the following steps:
step 1. delivering the crude oil to a first tube group of a convection section of a cracking furnace for preheating, and then performing vaporization to obtain a first gas phase and a first liquid phase;
step 2. performing high-pressure extraction on the first liquid phase to obtain a non-asphalt oil and an asphalt;
step 3. mixing the first gas phase and the non-asphalt oil with water vapor respectively, or mixing the first gas phase with the non-asphalt oil prior to mixing with water vapor, then delivering the same to a second tube group of the convection section of the cracking furnace for heating to a crossover temperature, followed by delivering the same to a radiation section of the cracking furnace for cracking to obtain a cracked product, and separating the cracked product to obtain low-carbon olefins.

In the present invention, the first gas phase obtained after vaporization of the crude oil is passed into the cracking furnace, and the non-asphalt oil (including an extracted oil) separated from the first liquid phase after the high-pressure extraction is passed into the cracking furnace. This method can effectively improve utilization rate and cracking efficiency of the crude oil, improve the yield of low-carbon olefins in the product, prolong the operating cycle, and reduce coking of the vaporized crude oil mixture during the cracking.

In the present invention, the crude oil includes light crude oil, medium crude oil, heavy crude oil and extra-heavy crude oil, such as a de-crude oil after dehydration and desalination. The relative density of the light crude oil at 20°C is <0.8661, the relative density of the medium crude oil at 20°C is 0.8661-0.9162, the relative density of the heavy crude oil at 20°C is 0.9162-0.9968, and the relative density of the extra-heavy crude oil at 20°C is >0.9968. Preferably, the crude oil in the present invention can be the medium crude oil, the heavy crude oil and the extra-heavy crude oil.

In a preferred embodiment, total content of colloids and asphaltenes in the crude oil is greater than 1 wt%, preferably, the total content of colloids and asphaltenes in the crude oil is greater than 5 wt%.

In the present invention, the crude oil is not limited to the above, and can also include oil obtained by primary processing of crude oil, oil obtained by secondary processing of crude oil, and coal-to-oil; preferably, the primary processing includes atmospheric distillation and/or vacuum distillation, the oil obtained by primary processing of crude oil includes light naphtha, naphtha, diesel oil, residual oil, etc.; preferably, the secondary processing includes thermal cracking, catalytic cracking, hydrocracking, delayed coking, and catalytic reforming, the oil obtained by secondary processing of crude oil includes hydrocracked tail oil, hydrogenated diesel oil, gasoline, raffinate oil, etc.

In a preferred embodiment, in step 1, an outflow temperature of the crude oil after preheating is about 120-350°C, preferably about 120-315°C, more preferably about 150-300°C, wherein the outflow temperature of the crude oil after preheating refers to the temperature at which crude oil flows out of the convection section of the cracking furnace after preheating in said convection section.

In a preferred embodiment, in step 1, a liquid content in the first gas phase is lower than 10 g/m³, preferably lower than 200 mg/m³.

In a preferred embodiment, in step 1, the vaporization is at least one of stripping, flash evaporation and cyclone separation, and cyclone separation is preferably used for the vaporization.

In a preferred embodiment, in step 1, a cyclone separator is used for the vaporization.

In a further preferred embodiment, the cyclone separator is selected from a volute cyclone separator, an axial guide vane cyclone separator, a cylindrical cyclone separator, a cone-cylinder combined cyclone separator or a direct current cyclone separator.

In a still further preferred embodiment, the cyclone separator is provided with internal members, including a skimming cartridge located at the top of the cyclone separator and/or a baffle and a vortex breaker in the lower part of the cyclone separator.

Herein, the cyclone separator has advantages of small volume and high separation efficiency.

In the present invention, "cyclone separator" refers to a cyclone separator in a broad sense, including not only a "cyclone separator" in a narrow sense, but also a separation device with a principle similar thereto in the art, such as a hydrocyclone separator. Unless otherwise specified, the cyclone separator mentioned in the present invention is the cyclone separator in a broad sense. According to the present invention, working media of the cyclone separator for vaporization are mostly gas, and a cyclone separator commonly used in the field can be selected, as long as a preheated crude oil can be separated at a temperature of 120-350°C, preferably 120-315°C and the separation efficiency can reach 90% or more.

Herein, the number of cyclone separators can be selected according to need, and one or more cyclone separators can be comprised. When multiple cyclone separators are comprised, the multiple cyclone separators can be connected in parallel or in series.

In one embodiment, in step 2, the high-pressure extraction includes extraction treatment, optional sedimentation separation treatment, and solvent recovery treatment. In the case of not undergoing the sedimentation separation treatment, the first liquid phase is subjected to the extraction treatment and solvent recovery treatment to obtain the asphalt and the non-asphalt oil, respectively. The asphalt is discharged externally, and the non-asphalt oil can be delivered to the cracking furnace for subsequent cracking. In the case of undergoing the sedimentation separation treatment, the first liquid phase is subjected to the extraction treatment, sedimentation separation treatment and solvent recovery treatment to obtain an extracted oil, a sedimented oil and the asphalt, respectively. The sedimented oil and the asphalt are discharged externally, and the extracted oil can be delivered to the cracking furnace for subsequent cracking.

It should be pointed out that, as to whether the first liquid phase needs to undergo the sedimentation separation treatment, it mainly depends on the crude oil feed. If the crude oil contains less heavy hydrocarbons, in the high-pressure extraction, the first liquid phase undergoes the extraction treatment to remove asphalt followed by directly performing the solvent recovery treatment to recover a solvent, and obtain the non-asphalt oil, which can be applied in cracking. If the crude oil contains a higher content of heavy hydrocarbons, after the first liquid phase undergoes the extraction treatment to remove asphalt, it further needs to undergo a sedimentation separation treatment to remove the sedimented oil, and then the material removed from the asphalt and the sedimented oil is subjected to the solvent recovery treatment to recover a solvent, and obtain the extracted oil, which can be applied in cracking.

In the present invention, "high pressure" generally refers to a pressure of about 0.5-40 MPa, preferably about 0.8-25 MPa, more preferably about 1-10 MPa.

In a preferred embodiment, in step 2, the first liquid phase is subjected to the high-pressure extraction to obtain the extracted oil, the sedimented oil and the asphalt.

In a preferred embodiment, in step 2, the first liquid phase is subjected to the high-pressure extraction to obtain the extracted oil, the sedimented oil and the asphalt, and in step 3, the first gas phase and the extracted oil are respectively mixed with water vapor, or the first gas phase is mixed with the extracted oil prior to being mixed with water vapor, followed by delivery of the same to a second tube group of the convection section of the cracking furnace for heating to the crossover temperature, followed by delivery to the radiation section of the cracking furnace for cracking to obtain the cracked product, and separation of the cracked product to obtain low-carbon olefins.

In a further preferred embodiment, in step 2, the first liquid phase is subjected to the high-pressure extraction to obtain the extracted oil, the sedimented oil and the asphalt, and in step 3, the first gas phase and the extracted oil are respectively mixed with water vapor, followed by delivery of the same to the second tube group of the convection section of the cracking furnace for heating to the crossover temperature, followed by delivery to the radiation section of the cracking furnace for cracking to obtain the cracked product, and separation of the cracked product to obtain low-carbon olefins. In a preferred embodiment, in step 2, the high-pressure extraction includes extraction treatment, sedimentation separation treatment and solvent recovery treatment, which are preferably carried out in an extraction column, a sedimentation column and a solvent recovery column, respectively.

In a further preferred embodiment, the extraction treatment is carried out in the presence of a solvent, preferably the solvent is a low-carbon hydrocarbon, more preferably, the low-carbon hydrocarbon includes but is not limited to at least one selected from the group consisting of propane, butane, pentane, propylene and butene. In a preferred embodiment, the high-pressure extraction comprises the following steps:
step (I) subjecting the first liquid phase and the solvent to extraction treatment to extract a first light phase component and an asphalt, wherein the first light phase component includes a non-asphalt portion and the solvent, and discharging the asphalt externally;
   wherein, preferably, the extraction treatment is carried out in an extraction column, more preferably, the first liquid phase is fed into the upper part of the extraction column, the solvent is fed into the lower part of the extraction column, the first light component including the non-asphalt portion and the solvent is obtained at the top of the column, and the asphalt is obtained at the bottom of the column and is discharged externally;
   wherein the solvent extracts the non-asphalt portion in the first liquid phase (which portion is preferably withdrawn from the top of the extraction column), and the asphalt is discharged from the bottom of the extraction column for subsequent treatment;
step (II) subjecting the first light component to a temperature increasing treatment and then to the sedimentation separation treatment to obtain a second light phase component and a sedimented oil, wherein the second light phase component includes the solvent and an extracted oil, and discharging the sedimented oil externally;
   wherein preferably, the sedimentation separation treatment is carried out in a precipitation column, more preferably, the second light component including the solvent and the extracted oil is withdrawn from the top of the sedimentation column, and the sedimented oil is obtained at the bottom of the sedimentation column and is discharged externally;
step (III) subjecting the second light component to a temperature increasing treatment and then to the solvent recovery treatment to obtain recovered solvent and the extracted oil.

Wherein, the solvent obtained in step (III) is recycled after being optionally cooled, and the extracted oil is delivered to a cracking furnace for subsequent cracking.

In a preferred embodiment, in step (I), a mass ratio of the solvent to the first liquid phase is about (0.1-20):1, preferably (1-10):1, e.g., 4:1.

In the high-pressure extraction of the present invention, the first light phase component including the non-asphalt portion and the solvent, which is extracted from the extraction treatment, is subjected to the temperature increasing treatment before the sedimentation separation treatment, and the second light phase component including the solvent and the extracted oil, which is obtained from the sedimentation separation treatment, is subjected to the temperature increasing treatment before the solvent recovery treatment, wherein the purpose of increasing temperature is to reduce density and solubility of the solvent, so that part or all of the crude oil components dissolved in the solvent are precipitated out.

In the present invention, the extraction treatment, the sedimentation separation treatment and the solvent recovery treatment are performed under high pressure. Preferably, pressures of the extraction treatment, the sedimentation separation treatment and the solvent recovery treatment are independently about 0.5-40 MPa, preferably about 0.8-25 MPa, more preferably about 1-10 Mpa; more preferably, the pressures of the extraction treatment, the sedimentation separation treatment and the solvent recovery process are the same or similar.

In a preferred embodiment, a temperature of the extraction treatment is about 30-300°C, preferably about 50-200°C.

In a preferred embodiment, a temperature of the sedimentation separation treatment is about 0.5-50°C higher, preferably about 1-30°C higher than that of the extraction treatment.

In a preferred embodiment, a temperature of the solvent recovery treatment is about 0.5-50°C higher, preferably about 1-30°C higher than that of the sedimentation separation treatment.

In a preferred embodiment, the high-pressure extraction includes a supercritical extraction.

In a preferred embodiment, the high-pressure extraction is carried out in a supercritical state, wherein the temperature and pressure conditions set during the extraction treatment in step (I) make the solvent in a supercritical or near-supercritical liquid phase region, the solvent extracts the non-asphalt portion in the first liquid phase (which is preferably withdrawn from the top of the extraction column), and the asphalt is discharged from the bottom of the extraction column for subsequent treatment.

In a preferred embodiment, the extraction treatment, the sedimentation separation treatment and the solvent recovery treatment are all carried out in a supercritical or near-supercritical state.

In a preferred embodiment, in the supercritical extraction treatment, the extraction treatment conditions are: a pressure of about 1-10MPa, preferably about 3-6MPa; and/or, a temperature of about 50-200°C, preferably about 80-160°C.

In a preferred embodiment, in the supercritical extraction treatment, the conditions for the sedimentation separation treatment are: a pressure of about 1-10 MPa, preferably about 3-6 MPa; and/or, a temperature of about 1-30°C higher, preferably about 3-8°C higher than that of the extraction treatment.

In a preferred embodiment, in the supercritical extraction treatment, the conditions for the solvent recovery treatment are: a pressure of about 1-10 MPa, preferably about 3-6 MPa; and/or, a temperature of about 1-30°C higher, preferably about 5-20°C higher than that of the sedimentation separation treatment.

Wherein, the pressures of the extraction treatment, the sedimentation separation treatment and the solvent recovery treatment are independently about 1-10MPa, preferably about 3-6Mpa; preferably the pressures of the extraction treatment, the sedimentation separation treatment and the solvent recovery treatment are the same or similar. In a preferred embodiment, the pressure and temperature conditions of the extraction treatment, the sedimentation separation treatment and the solvent recovery treatment are controlled to ensure that the three treatments are carried out in a supercritical or near-supercritical state.

In a preferred embodiment, step 3 includes the following substeps:
step 3.1 mixing the first gas phase with water vapor and then delivering the same to a first tube row of the second tube group in the convection section of the cracking furnace for heating to the crossover temperature, followed by delivery to a first radiation section of the cracking furnace for a first cracking to obtain a first cracked product;
step 3.2 mixing the non-asphalt oil with water vapor and then delivering the same to a second tube row of the second tube group in the convection section of the cracking furnace for heating to the crossover temperature, followed by delivery to a second radiation section of the cracking furnace for a second cracking to obtain a second cracked product;
step 3.3 separating the first cracked product and the second cracked product respectively or after mixing them, to obtain low-carbon olefins.

In a more preferred embodiment, step 3 includes the following substeps:
step 3.1 mixing the first gas phase with water vapor and then delivering the same to a first tube row of the second tube group in the convection section of the cracking furnace for heating to the crossover temperature, followed by delivery to a first radiation section of the cracking furnace for a first cracking to obtain a first cracked product;
step 3.2 mixing the extracted oil with water vapor and then delivering the same to a second tube row of the second tube group in the convection section of the cracking furnace for heating to the crossover temperature, followed by delivery to a second radiation section of the cracking furnace for a second cracking to obtain a second cracked product;
step 3.3 separating the first cracked product and the second cracked product respectively or after mixing them, to obtain low-carbon olefins.

In a preferred embodiment, in the present invention, the first gas phase and the non-asphalt oil, preferably the extracted oil, are respectively subjected to cracking in different environments. Specifically, the lightest first gas phase can be cracked at a relatively high temperature to increase the cracking degree to the maximum extent and increase the product yield; while the relatively heavy non-asphalt oil, preferably the extracted oil, is cracked at a relatively low temperature, which can reduce the coking degree.

In a preferred embodiment, the crossover temperature in step 3.1 is about 500-750°C, preferably about 540-700°C.

In a preferred embodiment, an outlet temperature of the first radiation section in step 3.1 is about 780-950°C, preferably about 800-900°C.

In a preferred embodiment, a residence time of the first cracking in step 3.1 is about 0.05-1s, preferably about 0.1-0.7 s.

In a preferred embodiment, a water-to-oil ratio of the first cracking in step 3.1 is about (0.1-2):1, preferably about (0.4-1.5):1.

Wherein, the water-to-oil ratio refers to the weight ratio.

In a preferred embodiment, the crossover temperature in step 3.2 is about 520-760°C, preferably about 550-710°C, more preferably lower than the crossover temperature in step 3.1.

In a preferred embodiment, an outlet temperature of the second radiation section in step 3.2 is about 750-950°C, preferably about 760-900°C, more preferably lower than that of the first radiation section in step 3.1.

In a preferred embodiment, a residence time of the second cracking in step 3.2 is about 0.05-1s, preferably about 0.1-0.7 s.

In a preferred embodiment, a water-to-oil ratio of the second cracking in step 3.2 is about (0.1-2):1, preferably about (0.4-1.5):1.

In a preferred embodiment, in step 3.3, the separation is performed as follows: the cracked product is sequentially delivered into a quencher and a cracked gas separation system to obtain products such as hydrogen, methane, ethylene, propylene, butadiene, pyrolysis tar oil, etc., and the separation process in the prior art can be used for the separation.

In a further preferred embodiment, the cracked gas is heat-exchanged prior to separation.

Herein, to further improve the yield of low-carbon olefins in the product, subjecting the cracked gas to heat exchange treatment prior to separation can further improve the yield of low-carbon olefins in the product and reduce coking of the cracked gas during cooling and separation.

Preferably, the water vapor in steps 3.1 and 3.2 is the superheated water vapor in the convection section of the cracking furnace.

In another aspect, the present invention provides a system for cracking crude oil, preferably for carrying out the method of the present invention, the system including a cracking furnace, a vaporization unit and a high-pressure extraction unit, and the cracking furnace including a convection section and a radiation section arranged in sequence along a fluid direction. The vaporization unit is provided thereon with a first gas phase outlet and a first liquid phase outlet.

In a preferred embodiment, the convection section forms a closed-loop connection with a material inlet of the vaporization unit and the first gas phase outlet of the vaporization unit.

In a preferred embodiment, the first liquid phase outlet of the vaporization unit is connected to the high-pressure extraction unit, for delivering the first liquid phase produced by the vaporization unit to the high-pressure extraction unit.

In a preferred embodiment, the high-pressure extraction unit includes an extraction unit, an optional sedimentation separation unit and a solvent recovery unit connected in sequence.

In a further preferred embodiment, the extraction unit, the sedimentation separation unit and the solvent recovery unit are all provided thereon with a light phase outlet and a heavy phase outlet.

In a preferred embodiment, the extraction unit is provided thereon with a solvent inlet and a first liquid phase inlet, the first liquid phase inlet is connected to the first liquid phase outlet of the vaporization unit, and the solvent inlet is connected to the light phase outlet of the solvent recovery unit.

In a preferred embodiment, the high-pressure extraction unit includes a sedimentation separation unit, and the light phase outlet of the extraction unit is connected to a material inlet of the sedimentation separation unit.

In a preferred embodiment, the high-pressure extraction unit includes a sedimentation separation unit, and the light phase outlet of the sedimentation separation unit is connected to a material inlet of the solvent recovery unit.

In a preferred embodiment, the heavy phase outlet of the solvent recovery unit and the first gas phase outlet of the vaporization unit are connected respectively or after being combined, to a material inlet of the convection section of the cracking furnace .

In a preferred embodiment, the vaporization unit is a cyclone separator, preferably, the cyclone separator is selected from a rectangular inlet cyclone separator, a volute cyclone separator, an axial guide vane cyclone separator, a cylindrical cyclone separator, a cone-cylinder combined cyclone separator, a counter-current cyclone separator, a direct-current cyclone separator or a direct-current multi-cyclone tubes separator; preferably a volute cyclone separator, an axial guide vane cyclone separator, a cylindrical cyclone separator, a cone-cylinder combined cyclone separator, or a direct-current cyclone separator. The cyclone separator is provided with internal members, including a skimming cartridge located at the top of the cyclone separator and/or a baffle and a vortex breaker located in the lower part of the cyclone separator.

In a preferred embodiment, the system further includes a cracked product separation unit for separating the cracked product obtained via the cracking furnace.

In a preferred embodiment of the present invention, the crude oil is delivered to the first tube group of the convection section of the cracking furnace for preheating, and thereafter separated by the vaporization unit to form the first gas phase and the first liquid phase; the first gas phase is delivered to the first tube row of the second tube group in the convection section of the cracking furnace; the first liquid phase is delivered to the high-pressure extraction unit, wherein the first liquid phase is sequentially processed by the extraction unit, the sedimentation separation unit and the solvent recovery unit, and at the same time, a solvent is introduced to the high-pressure extraction unit. The asphalt is discharged externally from the extraction unit, the sedimented oil is discharged externally from the sedimentation separation unit, and from the solvent recovery unit a gas phase solvent is withdrawn and recycled back to the extraction unit, and the extracted oil is withdrawn and delivered to the second tube row of the second tube group in the convection section of the cracking furnace. The first gas phase and the extracted oil are respectively cracked in the first radiation section and the second radiation section of the cracking furnace to obtain a cracked product, which is processed by the cracked product separation unit to obtain low-carbon olefins.

In a preferred embodiment, the high-pressure extraction unit may include a supercritical extraction treatment unit.

Compared with the prior art, the beneficial effects of the present invention include but are not limited to the following: in the present invention, after the crude oil is preheated in the cracking furnace and vaporized, the obtained first gas phase is passed into the cracking furnace, the obtained first liquid phase is passed into the high-pressure extraction unit, and the non-asphalt oil, preferably the extracted oil, separated via the high-pressure extraction is passed into the cracking furnace, thus allowing colloids and asphaltenes in the crude oil to be effectively removed, so that the non-asphalt oil (including the extracted oil) is substantially free of colloids and asphaltenes, thereby improving the utilization rate and cracking efficiency of the crude oil, improving the yield of low-carbon olefins in the product, prolonging the operating cycle to 42 days or more, and reducing coking of the gasified crude oil mixture during cracking.

### BRIEF DESCRIPTION OF DRAWINGS

Figure 1 shows a schematic structural diagram of the system of the present invention.
Figure 2 shows a schematic structural diagram of the system of the present invention.
Figure 3 shows a schematic structural diagram of the system adopted in Example 5.

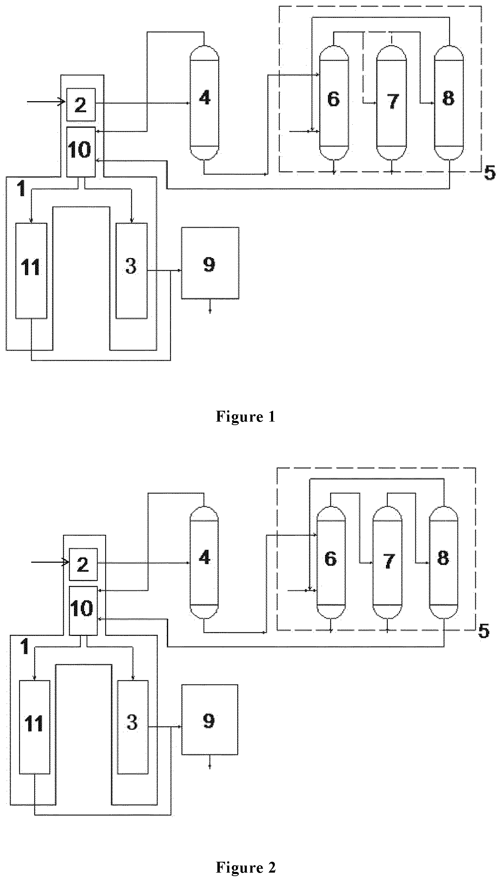

In Figure 1, Figure 2 and Figure 3 are present: 1 - a cracking furnace, 2 - a first tube group of a convection section, 3 - a first radiation section, 4 - a vaporization unit, 5 - a high-pressure extraction unit, 6 - an extraction unit, 7 - a sedimentation separation unit, 8 - a solvent recovery unit; 9 - a cracked product separation unit; 10 - a second tube group of the convection section; 11 - a second radiation section.

In Figure 1, crude oil is delivered to the first tube group 2 of the convection section of the cracking furnace 1 for preheating, and thereafter separated by the vaporization unit 4 to form a first gas phase and a first liquid phase; the first gas phase is delivered to the first tube row of the second tube group 10 in the convection section of the cracking furnace 1 for heating to the crossover temperature and then passed into the first radiation section 3 for a first cracking to obtain a first cracked product; the first liquid phase is delivered to the high-pressure extraction unit 5, wherein the first liquid phase is sequentially processed by the extraction unit 6, optionally the sedimentation separation unit 7 and the solvent recovery unit 8, and at the same time, a solvent is introduced to the high-pressure extraction unit 5. When a sedimentation separation treatment is not required, the first liquid phase is sequentially processed by the extraction unit 6 and the solvent recovery unit 8, but not by the sedimentation separation unit 7. An asphalt is discharged externally from the extraction unit 6, and from the solvent recovery unit 8, a gas phase solvent is withdrawn and recycled back to the extraction unit 5, and a non-asphalt oil is withdrawn and delivered to the second tube row of the second tube group 10 in the convection section of the cracking furnace 1 for heating to the crossover temperature, and then passed into the second radiation section 11 for the second cracking to obtain a second cracked product. The first cracked product and the second cracked product are processed by the cracked product separation unit 9 to obtain low-carbon olefins. When a sedimentation separation treatment is required, the first liquid phase is processed by the sedimentation separation unit 7, as shown in Fig. 2 below for details.

In Figure 2, crude oil is delivered to the first tube group 2 of the convection section of the cracking furnace 1 for preheating, and thereafter separated by the vaporization unit 4 to form a first gas phase and a first liquid phase; the first gas phase is delivered to the first tube row of the second tube group 10 in the convection section of the cracking furnace 1 for heating to the crossover temperature and then passed into the first radiation section 3 for a first cracking to obtain a first cracked product; the first liquid phase is delivered to the high-pressure extraction unit 5, wherein the first liquid phase is sequentially processed by the extraction unit 6, the sedimentation separation unit 7 and the solvent recovery unit 8, and at the same time, a solvent is introduced to the high-pressure extraction unit 5. An asphalt is discharged externally from the extraction unit 6, a sedimented oil is discharged externally from the sedimentation separation unit 7, and from the solvent recovery unit 8, a gas phase solvent is withdrawn and recycled back to the extraction unit 5, and an extracted oil is withdrawn and delivered to the second tube row of the second tube group 10 in the convection section of the cracking furnace 1 for heating to the crossover temperature, and then passed into the second radiation section 11 for a second cracking to obtain a second cracked product. The first cracked product and the second cracked product are processed by the cracked product separation unit 9 to obtain low-carbon olefins.

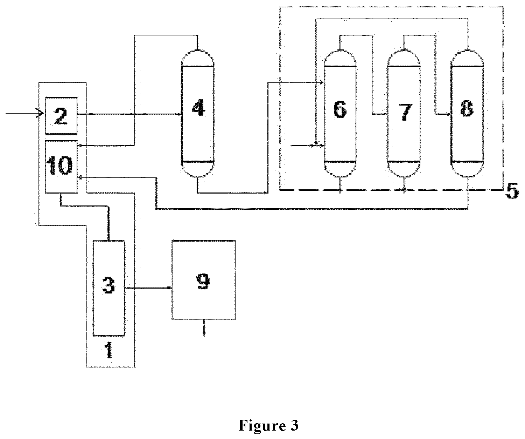

In Figure 3, crude oil is delivered to the first tube group 2 of the convection section of the cracking furnace 1 for preheating, and thereafter separated by the vaporization unit 4 to form a first gas phase and a first liquid phase; the first gas phase is delivered to the second tube group 10 in the convection section of the cracking furnace 1; the first liquid phase is delivered to the high-pressure extraction unit 5, wherein the first liquid phase is sequentially processed by the extraction unit 6, the sedimentation separation unit 7 and the solvent recovery unit 8, and at the same time, a solvent is introduced to the high-pressure extraction unit 5. An asphalt is discharged externally from the extraction unit 6, a sedimented oil is discharged from the sedimentation separation unit 7, and from the solvent recovery unit 8, a gas phase solvent is withdrawn and recycled back to the extraction unit 5, and an extracted oil is withdrawn and delivered to the second tube group 10 in the convection section of the cracking furnace 1. The first gas phase and the extracted oil are cracked in the first radiation section 3 of the cracking furnace 1 (herein the cracking furnace 1 comprises the first radiation section 3 only) to obtain a cracked product, which is processed by the cracked product separation unit 9 to obtain low-carbon olefins.

### EXAMPLES

The present invention will be specifically described below in combination with specific examples. It is necessary to point out that the following examples are only used to further illustrate the present invention, and should not be construed as limitations on the protection scope of the present invention. Some non-essential improvements and adjustments made by a person skilled in the art to the present invention according to the contents of the invention still belong to the protection scope of the present invention.

In addition, it should be noted that various specific technical features described in the following specific examples may be combined in any suitable manner under the circumstance that there is no contradiction. In order to avoid unnecessary repetition, the present invention will not describe the various possible combinations.

The cracking furnace used in Examples 1-4 of the present invention is a double-radiation section cracking furnace (a double-hearth cracking furnace), specifically a CBL-VII model cracking furnace (purchased from Sinopec Group Corporation). The cracking furnace used in Example 5 of the present invention and Comparative Example is a single-radiation section cracking furnace (a single-hearth cracking furnace), specifically a CBL-III model cracking furnace (purchased from Sinopec Group Corporation).

The crude oil used in the following examples and comparative example has a relative density of 0.8724 (20°C), a colloid content of 8.8 wt%, and an asphaltene content of 0.2 wt%, and distillation range analysis shown in Table 1, as measured according to the ASTM D5307 method.

**Table 1**

| Distillate mass fraction/% | Temperature/°C |
|---|---|
| Initial boiling point | 69 |
| 10 | 192 |
| 20 | 272 |
| 30 | 337 |
| 40 | 393 |
| 50 | 446 |
| 60 | 508 |
| 70 | 595 |
| 75 | 653 |
| 80 | - |
| 90 | - |
| Final boiling point | - |

### Example 1

The example was carried out by using the system shown in Figure 2, wherein a guide vane type cyclone separator with internal members was used as the vaporization unit, the extraction unit 6 was an extraction column, the sedimentation separation unit 7 was a sedimentation column, and the solvent recovery unit 8 was a solvent recovery column.
(1) Dehydrated and desalted crude oil was preheated to 288°C in the convection section 2 of the cracking furnace 1 to become a preheated crude oil with a pressure of 0.12 MPa. The preheated crude oil was delivered to the cyclone separator for vaporization to obtain a first gas phase and a first liquid phase, wherein a liquid content in the first gas phase was 270 mg/m³, a content of colloids in the first liquid phase was 11.3 wt%, and a content of asphaltenes in the first liquid phase was 0.26 wt%.
(2) The first liquid phase was delivered to the upper part of the extraction column, a solvent n-butane was passed into the lower part of the extraction column, a mass ratio of the solvent to the first liquid phase was 4:1, a pressure of the extraction column was 4 MPa, and a temperature of the extraction column was 160°C. A non-asphalt portion in the first liquid phase was extracted with the solvent and withdrawn from the top of the extraction column, and an asphalt was discharged from the bottom of the column. The solvent and the non-asphalt portion withdrawn from the top of the extraction column were subjected to temperature increase to 165°C and then passed into the sedimentation column, the solvent and an extracted oil were withdrawn from the top of the sedimentation column, and a sedimented oil was withdrawn from the bottom of the sedimentation column. The solvent and the extracted oil withdrawn from the top of the sedimentation column were subjected to temperature increase to 180°C and passed into the solvent recovery column, where the solvent and the extracted oil were separated, the solvent was withdrawn from the top of the column and then subjected to temperature decrease to 160°C for recycling, and the extracted oil was withdrawn from the bottom of the column. No colloids and asphaltenes were detected in the extracted oil (by an analysis method of SY/T 7550-2000).
(3) After the first gas phase was mixed with water vapor (water-to-oil ratio of 0.7), the mixture was delivered to the first tube row of the second tube group in the convection section of the cracking furnace, heated to the crossover temperature (600°C), and then directly delivered to the first radiation section for a first cracking to obtain a first cracked product. Operating parameters of the first radiation section 3 were a furnace tube outlet temperature of 810°C and a residence time of 0.22 s.
(4) After the extracted oil was mixed with water vapor (water-to-oil ratio of 0.75), the mixture was delivered to the second tube row of the second tube group in the convection section of the cracking furnace, heated to the crossover temperature (575°C), and then directly delivered to the second radiation section for a second cracking to obtain a second cracked product. Operating parameters of the second radiation section 11 were a furnace tube outlet temperature of 770°C and a residence time of 0.21s.
(5) The first cracked product and the second cracked product were mixed and then passed through a quenching unit and a separation unit for separating a cracked product (using a sequential separation process of LUMMUS) to be separated to obtain low-carbon olefins.

The cracked gas was separated to obtain low-carbon olefins, with the yield of ethylene being 25.07 wt%, the yield of propylene being 13.79 wt%, the yield of 1,3-butadiene being 4.66 wt%, and the yield of triene being 43.52 wt%. The operating cycle was 50 days.

### Example 2

The example was carried out by using the system shown in Figure 2, wherein a guide vane type cyclone separator with internal members was used as the vaporization unit, the extraction unit 6 was an extraction column, the sedimentation separation unit 7 was a sedimentation column, and the solvent recovery unit 8 was a solvent recovery column.
(1) Dehydrated and desalted crude oil was preheated to 230°C in the convection section 2 of the cracking furnace 1 to become a preheated crude oil with a pressure of 0.13 MPa. The preheated crude oil was delivered to the cyclone separator for vaporization to obtain a first gas phase and a first liquid phase, wherein a liquid content in the first gas phase was 150 mg/m³, a content of colloids in the first liquid phase was 10.2 wt%, and a content of asphaltenes in the first liquid phase was 0.33 wt%.
(2) The first liquid phase was delivered to the upper part of the extraction column of the supercritical extraction unit, a solvent mixed C4 (n-butane and iso-butane of 50% for each) was passed into the lower part of the extraction column, a mass ratio of the solvent to the first liquid phase was 3:1, a pressure of the extraction column was 4 MPa, a temperature of the extraction column was 150°C. A non-asphalt portion in the first liquid phase was extracted with the solvent and withdrawn from the top of the extraction column, an asphalt was discharged from the bottom of the column. The solvent and the non-asphalt portion withdrawn from the top of the extraction column were subjected to temperature increase to 162°C and then passed into the sedimentation column, the solvent and an extracted oil were withdrawn from the top of the sedimentation column, and a sedimented oil was withdrawn from the bottom of the sedimentation column. The solvent and the extracted oil withdrawn from the top of the sedimentation column were subjected to temperature increase to 180°C and passed into the solvent recovery column, where the solvent and the extracted oil were separated, the solvent was withdrawn from the top of the column and then subjected to temperature decrease to 150°C for recycling, and the extracted oil was withdrawn from the bottom of the column. No colloids and asphaltenes were detected in the extracted oil (by an analysis method of SY/T 7550-2000).
(3) After the first gas phase was mixed with water vapor (water-to-oil ratio of 0.65), the mixture was delivered to the first tube row of the second tube group in the convection section of the cracking furnace, heated to the crossover temperature (610°C), and then directly delivered to the first radiation section for a first cracking to obtain a first cracked product. Operating parameters of the first radiation section 3 were a furnace tube outlet temperature of 815°C and a residence time of 0.25 s.
(4) After the extracted oil was mixed with water vapor (water-to-oil ratio of 0.65), the mixture was delivered to the second tube row of the second tube group in the convection section of the cracking furnace, heated to the crossover temperature (585°C), and then directly delivered to the second radiation section for a second cracking to obtain a second cracked product. Operating parameters of the second radiation section 11 were a furnace tube outlet temperature of 785°C and a residence time of 0.2 s.
(5) The first cracked product and the second cracked product were mixed and then passed through a quenching unit and a separation unit for separating a cracked product (using a sequential separation process of LUMMUS) to be separated to obtain low-carbon olefins.

The cracked product was separated to obtain low-carbon olefins, with the yield of ethylene being 25.31 wt%, the yield of propylene being 13.41 wt%, the yield of 1,3-butadiene being 4.73 wt%, and the yield of triene being 43.46 wt%. The operating cycle was 52 days.

### Example 3

The example was carried out by using the system shown in Figure 2, wherein a guide vane type cyclone separator with internal members was used as the vaporization unit, the extraction unit 6 was an extraction column, the sedimentation separation unit 7 was a sedimentation column, and the solvent recovery unit 8 was a solvent recovery column.
(1) Dehydrated and desalted crude oil was preheated to 315°C in the convection section 2 of the cracking furnace 1 to become a preheated crude oil with a pressure of 0.12 MPa. The preheated crude oil was delivered to the cyclone separator for vaporization to obtain a first gas phase and a first liquid phase, wherein a liquid content in the first gas phase was 200 mg/m³, a content of colloids in the first liquid phase was 11.9 wt%, and a content of asphaltenes in the first liquid phase was 0.27 wt%.
(2) The first liquid phase was delivered to the upper part of the extraction column, a solvent n-butane was passed into the lower part of the extraction column, a mass ratio of the solvent to the first liquid phase was 8:1, a pressure of the extraction column was 3 MPa, and a temperature of the extraction column was 140°C. A non-asphalt portion in the first liquid phase was extracted with the solvent and withdrawn from the top of the extraction column, and an asphalt was discharged from the bottom of the column. The solvent and the non-asphalt portion withdrawn from the top of the extraction column were subjected to temperature increase to 148°C and then passed into the sedimentation column, the solvent and an extracted oil were withdrawn from the top of the sedimentation column, and a sedimented oil was withdrawn from the bottom of the sedimentation column. The solvent and the extracted oil withdrawn from the top of the sedimentation column were subjected to temperature increase to 180°C and passed into the solvent recovery column, where the solvent and the extracted oil were separated, the solvent was withdrawn from the top of the column and then subjected to temperature decrease to 140°C for recycling, and the extracted oil was withdrawn from the bottom of the column. No colloids and asphaltenes were detected in the extracted oil (by an analysis method of SY/T 7550-2000).
(3) After the first gas phase was mixed with water vapor (water-to-oil ratio of 0.80), the mixture was delivered to the first tube row of the second tube group in the convection section of the cracking furnace, heated to the crossover temperature (600°C), and then directly delivered to the first radiation section for a first cracking to obtain a first cracked product. Operating parameters of the first radiation section 3 were a furnace tube outlet temperature of 800°C and a residence time of 0.28 s.
(4) After the extracted oil was mixed with water vapor (water-to-oil ratio of 1), the mixture was delivered to the second tube row of the second tube group in the convection section of the cracking furnace, heated to the crossover temperature (570°C), and then directly delivered to the second radiation section for a second cracking to obtain a second cracked product. Operating parameters of the second radiation section 11 were a furnace tube outlet temperature of 765°C and a residence time of 0.15 s.
(5) The first cracked product and the second cracked product were mixed and then passed through a quenching unit and a separation unit for separating a cracked product (using a sequential separation process of LUMMUS) to be separated to obtain low-carbon olefins.

The cracked gas was separated to obtain low-carbon olefins, with the yield of ethylene being 22.85 wt%, the yield of propylene being 12.57 wt%, the yield of 1,3-butadiene being 4.27 wt%, and the yield of triene being 39.69 wt%. The operating cycle was 48 days.

### Example 4

The example was carried out by using the system shown in Figure 2, wherein a guide vane type cyclone separator with internal members was used as the vaporization unit, the extraction unit 6 was an extraction column, the sedimentation separation unit 7 was a sedimentation column, and the solvent recovery unit 8 was a solvent recovery column.
(1) Dehydrated and desalted crude oil was preheated to 200°C in the convection section 2 of the cracking furnace 1 to become a preheated crude oil with a pressure of 0.12 MPa. The preheated crude oil was delivered to the cyclone separator for vaporization to obtain a first gas phase and a first liquid phase, wherein a liquid content in the first gas phase was 100 mg/m³, a content of colloids in the first liquid phase was 9.9 wt%, and a content of asphaltenes in the first liquid phase was 0.22 wt%.
(2) The first liquid phase was delivered to the upper part of the extraction column, a solvent n-butane was passed into the lower part of the extraction column, a mass ratio of the solvent to the first liquid phase was 10:1, a pressure of the extraction column was 18 MPa, and a temperature of the extraction column was 160°C. A non-asphalt portion in the first liquid phase was extracted with the solvent and withdrawn from the top of the extraction column, and an asphalt was discharged from the bottom of the column. The solvent and the non-asphalt portion withdrawn from the top of the extraction column were subjected to temperature increase to 170°C and then passed into the sedimentation column, the solvent and an extracted oil were withdrawn from the top of the sedimentation column, and a sedimented oil was withdrawn from the bottom of the sedimentation column. The solvent and extracted oil withdrawn from the top of the sedimentation column were subjected to temperature increase to 180°C and passed into the solvent recovery column, where the solvent and the extracted oil were separated, the solvent was withdrawn from the top of the column and then subjected to temperature decrease to 160°C for recycling, and the extracted oil was withdrawn from the bottom of the column. No colloids and asphaltenes were detected in the extracted oil (by an analysis method of SY/T 7550-2000).
(3) After the first gas phase was mixed with water vapor (water-to-oil ratio of 0.6), the mixture was delivered to the first tube row of the second tube group in the convection section of the cracking furnace, heated to the crossover temperature (620°C), and then directly delivered to the first radiation section for a first cracking to obtain a first cracked product. Operating parameters of the first radiation section 3 were a furnace tube outlet temperature of 820°C and a residence time of 0.2 s.
(4) After the extracted oil was mixed with water vapor (water-to-oil ratio of 0.8), the mixture was delivered to the second tube row of the second tube group in the convection section of the cracking furnace, heated to the crossover temperature (590°C), and then directly delivered to the second radiation section for a second cracking to obtain a second cracked product. Operating parameters of the second radiation section 11 were a furnace tube outlet temperature of 790°C and a residence time of 0.21s.
(5) The first cracked product and the second cracked product were mixed and then passed through a quenching unit and a separation unit for separating a cracked product (using a sequential separation process of LUMMUS) to be separated to obtain low-carbon olefins.

The cracked gas was separated to obtain low-carbon olefins, with the yield of ethylene being 23.51 wt%, the yield of propylene being 14.58 wt%, the yield of 1,3-butadiene being 4.35 wt%, and the yield of triene being 42.44 wt%. The operating cycle was 49 days.

### Example 5

The example was carried out by using the system shown in Figure 3, wherein a cyclone separator was used as the vaporization unit, and the cracking furnace comprised the first radiation section 3 only.

Steps (1) to (2) of Example 1 were repeated, and the difference lied in that the first gas phase and the extracted oil were cracked together, as described in details in the following step (3):
(3) The first gas phase and the extracted oil were mixed with water vapor (water-to-oil ratio of 0.7), respectively, then the mixtures were delivered to the convection section of the cracking furnace, heated to the crossover temperature (585°C), and then directly delivered to the first radiation section 3 for cracking to obtain a cracked product. Operating parameters of the first radiation section 3 were a furnace tube outlet temperature of 780°C and a residence time of 0.22s. The cracked product was passed through a quenching unit and a separation unit for separating a cracked product (using a sequential separation process of LUMMUS) to be separated to obtain low-carbon olefins.

The cracked gas was separated to obtain low-carbon olefins, wherein the product yield was slightly lower than that of Example 1, with the yield of ethylene being 24.25 wt%, the yield of propylene being 12.61 wt%, the yield of 1,3-butadiene being 4.51 wt%, and the yield of triene being 41.37wt%, while the operating cycle was shorter than that of Example 1, and was only 42 days.

### Comparative Example 1

In this comparative example, a conventional cracking furnace was used for cracking.

Dehydrated and desalted crude oil was directly delivered to the cracking furnace, passed through the convection section to be heated to the crossover temperature (580°C), and then directly passed into the radiation section for cracking. The cracking conditions were a water-to-oil ratio of 0.75, an outlet temperature of the radiation section of 790 °C, an outlet pressure of the radiation section of 0.11 MPa, and a residence time of 0.22 s. The sequential separation process of LUMMUS was used for the cracked gas. Analysis of the steam cracking reaction product showed that the yield of ethylene was 21.49 wt%, the yield of propylene was 13.29 wt%, the yield of 1,3-butadiene was 4.03 wt%, and the yield of triene was 38.81 wt%. The operating cycle was 5 days.

From the results of the Examples and Comparative Example 1, it can be seen that using a traditional cracking apparatus, the crude oil was used to maintain only a 5-day operating cycle, while the present invention can ensure that the crude oil was normally used in the cracking apparatus, and use of the vaporization unit and the high-pressure extraction unit to treat the crude oil can effectively reduce the occurrence of coking and achieve an operating cycle of 42 days or more. Further, under the same process conditions, the examples effectively improved the yield of the low-carbon olefins as compared with the comparative example.

## Claims

1. Method for cracking crude oil, comprising the following steps:
step 1. delivering the crude oil to a first tube group of a convection section of a cracking furnace for preheating, and then performing vaporization to obtain a first gas phase and a first liquid phase;
step 2. performing high-pressure extraction on the first liquid phase to obtain a non-asphalt oil and an asphalt;
step 3. mixing the first gas phase and the non-asphalt oil with water vapor respectively, or mixing the first gas phase with the non-asphalt oil prior to mixing with water vapor, then delivering the same to a second tube group of the convection section of the cracking furnace for heating to crossover temperature, followed by delivering the same to a radiation section of the cracking furnace for cracking to obtain a cracked product, and separating the cracked product to obtain low-carbon olefins.

2. The method according to claim 1, **characterized in that**
in step 1, the vaporization is at least one of stripping, flash evaporation and cyclone separation, and cyclone separation is preferably used for the vaporization; and/or
in step 2, the high-pressure extraction includes an extraction treatment, an optional sedimentation separation treatment and a solvent recovery treatment; preferably, the high-pressure extraction includes a supercritical extraction.

3. The method according to claim 1 or 2, **characterized in that**
in step 2, the first liquid phase is subjected to the high-pressure extraction to obtain an extracted oil, a sedimented oil and the asphalt, and
in step 3, the first gas phase and the extracted oil are mixed with the water vapor respectively, or the first gas phase is mixed with the extracted oil prior to being mixed with the water vapor, followed by delivery of the same to the second tube group of the convection section of the cracking furnace for heating to the crossover temperature, followed by delivery to the radiation section of the cracking furnace for cracking to obtain the cracked product, and separation of the cracked product to obtain the low-carbon olefins.

4. The method according to claim 3, **characterized in that** step 3 comprises the following substeps:
step 3.1 mixing the first gas phase with the water vapor and then delivering the same to a first tube row of the second tube group in the convection section of the cracking furnace for heating to the crossover temperature, followed by delivery to a first radiation section of the cracking furnace for a first cracking to obtain a first cracked product;
step 3.2 mixing the extracted oil with the water vapor and then delivering the same to a second tube row of the second tube group in the convection section of the cracking furnace for heating to the crossover temperature, followed by delivery to a second radiation section of the cracking furnace for a second cracking to obtain a second cracked product;
step 3.3 separating the first cracked product and the second cracked product respectively or after mixing them, to obtain low-carbon olefins.

5. The method according to any one of claims 1 to 4, **characterized in that** in step 1,
an outflow temperature of the crude oil after preheating is 120-350°C, preferably 120-315°C, more preferably 150-300°C, and/or
a liquid content in the first gas phase is lower than 10 g/m³, preferably lower than 200 mg/m³.

6. The method according to claim 3, **characterized in that**
in step 2, the high-pressure extraction includes the extraction treatment, the sedimentation separation treatment and the solvent recovery treatment, which are preferably carried out in an extraction column, a sedimentation column and a solvent recovery column, respectively; preferably, the extraction treatment is carried out in the presence of a solvent, preferably the solvent is a low-carbon hydrocarbon, more preferably, the low-carbon hydrocarbon is at least one selected from the group consisting of propane, butane, pentane, propylene and butene.

7. The method according to claim 6, **characterized in that** the high-pressure extraction is carried out in a supercritical state, and comprises the following steps:
step (I) subjecting the first liquid phase and the solvent to the extraction treatment to extract a first light phase component and the asphalt, wherein the first light phase component includes a non-asphalt portion and the solvent, and discharging the asphalt externally;
preferably, in step (I), a mass ratio of the solvent to the first liquid phase is (0.1-20):1, preferably (1-10):1;
step (II) subjecting the first light component to a temperature increasing treatment and then to the sedimentation separation treatment to obtain a second light phase component and the sedimented oil, wherein the second light phase component includes the solvent and the extracted oil, and discharging the sedimented oil;
step (III) subjecting the second light component to the temperature increasing treatment and then to the solvent recovery treatment to obtain a recovered solvent and the extracted oil.

8. The method according to claim 7, **characterized in that**
pressures of the extraction treatment, the sedimentation separation treatment and the solvent recovery treatment are independently 1-10 MPa, preferably 3-6 MPa; and/or
a temperature of the extraction treatment is 50-200°C, preferably 80-160°C; and/or
a temperature of the sedimentation separation treatment is 1-30°C higher, preferably 3-8°C higher than that of the extraction treatment; and/or
a temperature of the solvent recovery treatment is 1-30°C higher, preferably 5-20°C higher than that of the sedimentation separation treatment.

9. The method according to any one of claims 4 to 8, **characterized in that**
the crossover temperature in step 3.1 is 500-750°C, preferably 540-700°C; and/or
an outlet temperature of the first radiation section in step 3.1 is 780-950°C, preferably 800-900°C; and/or
a residence time of the first cracking in step 3.1 is 0.05-1s, preferably 0.1-0.7 s; and/or
a water-to-oil ratio of the first cracking in step 3.1 is (0.1-2):1, preferably (0.4-1.5):1.

10. The method according to any one of claims 4 to 9, **characterized in that**
the crossover temperature in step 3.2 is 520-760°C, preferably 550-710°C; and/or
an outlet temperature of the second radiation section in step 3.2 is 750-950°C, preferably 760-900°C; and/or
a residence time of the second cracking in step 3.2 is 0.05-1s, preferably 0.1-0.7 s; and/or
a water-to-oil ratio of the second cracking in step 3.2 is (0.1-2):1, preferably (0.4-1.5):1.

11. System for cracking crude oil, for carrying out the method according to any one of claims 1 to 10, the system including a cracking furnace, a vaporization unit and a high-pressure extraction unit, wherein the cracking furnace includes a convection section and a radiation section arranged in sequence along a fluid direction, the high-pressure extraction unit includes an extraction unit, an optional sedimentation separation unit and a solvent recovery unit connected in sequence; preferably, the extraction unit, the sedimentation separation unit and the solvent recovery unit are all provided thereon with a light phase outlet and a heavy phase outlet, and the vaporization unit is provided thereon with a first gas phase outlet and a first liquid phase outlet.

12. The system according to claim 11, **characterized in that**
the extraction unit is provided thereon with a solvent inlet and a first liquid phase inlet, the first liquid phase inlet is connected to the first liquid phase outlet of the vaporization unit, and the solvent inlet is connected to the light phase outlet of the solvent recovery unit; and/or
the high-pressure extraction unit includes a sedimentation separation unit, and the light phase outlet of the extraction unit is connected to a material inlet of the sedimentation separation unit; and/or
the light phase outlet of the sedimentation separation unit is connected to a material inlet of the solvent recovery unit; and/or
the heavy phase outlet of the solvent recovery unit and the first gas phase outlet of the vaporization unit are connected respectively or after being combined, to a material inlet of the convection section of the cracking furnace.
